# EUROPÄISCHE PATENTSCHRIFT

(11) **EP 1 257 155 B1**
(45) Veröffentlichungstag und Bekanntmachung des Hinweises auf die Patenterteilung: **29.08.2007**
(21) Anmeldenummer: 02100443.7
(22) Anmeldetag: 06.05.2002
(51) Int. Cl.: H05G 1/46, H05G 1/44, H05G 1/32

(54) **Verfahren und Vorrichtung zur Belichtung von Röntgenaufnahmen**
Method and apparatus for the exposure of radiographs
Procedé et dispositif d'exposition des radiographies

(30) Priorität: 07.05.2001 DE 10122041
(43) Veröffentlichungstag der Anmeldung: 13.11.2002
(73) Patentinhaber: Philips Intellectual Property & Standards GmbH, 20099 Hamburg (DE); Koninklijke Philips Electronics N.V., 5621 BA Eindhoven (NL)
(72) Erfinder: Brendler, Joachim, 52066, Aachen (DE); Allmendinger, Horst, 52066, Aachen (DE)
(74) Vertreter: Damen, Daniel Martijn

(56) Entgegenhaltungen:
- EP-A- 0 063 644
- US-A- 4 097 741
- US-A- 4 956 857

## Beschreibung

Die Erfindung betrifft ein Verfahren zur Belichtung von Röntgenaufnahmen, insbesondere von Aufnahmen bei dynamischen Röntgenuntersuchungen eines Objektes, das heißt Untersuchungen, bei denen sich die Röntgenstrahlenabsorption des Objektes verändert und /oder das Objekt bewegt. Die Erfindung betrifft ferner einen Röntgengenerator mit einer Belichtungsautomatik zur Durchführung dieses Verfahrens.

Zur Röntgsnuntersuchung des menschlichen Körpers und seiner Organe sind an einem Röntgengenerator zahlreiche Einstellungen vorzunehmen, um eine optimale Aufnahme des Untersuchungsbereiches zu erzielen. Dies beruht darauf, dass die Dichte der verschiedenen Organe bzw. Körperbereiche an sich sehr unterschiedlich ist und sich auch bei verschiedenen Menschen in Abhängigkeit von deren Größe und Gewicht unterscheidet. Um eine für einen Patienten gefahrlose Untersuchung mit möglichst geringer Strahlenbelastung zu gewährleisten, sind weiterhin in fast allen Ländern gesetzliche Regelungen erlassen worden, aufgrund derer bestimmte Parameter nur innerhalb bestimmter Grenzen eingestellt bzw. verändert werden dürfen.

Insbesondere ist den folgenden, voneinander abhängigen und aufeinander abzustimmenden Parametern, die das aufgenommene Bild jeweils in unterschiedlicher Weise beeinflussen, besondere Beachtung zu schenken:

Zum einen bestimmt die Dosisleistung der Röntgenröhre (das heißt im wesentlichen die Aumahme-kV-Spannung) den Kontrast und den Kontrastumfang der abgebildeten Objekte. Die Strahlendosis bestimmt hingegen in erster Linie das Signal-Rausch-Verhältnis des Bildes, während die Belichtungszeit (Aufnahmezeit) insbesondere bei bewegten Objekten zur Optimierung der Bildschärfe einen bestimmten maximalen Wert nicht übersteigen darf. Zur Abstimmung bzw. Wahl dieser Parameter muss schließlich auch die Dichte (Röntgenstrahlenabsorption) des darzustellenden Objektes, das heißt im allgemeinen die Dicke des Patienten, berücksichtigt werden. Schließlich gelten auch für die auf einen Strahlungsempfänger auftreffende Strahlendosis oftmals verschiedene gesetzliche Bestimmungen oder Leitlinien.

Zur teilweisen Automatisierung der Einstellung dieser Parameter sind verschiedene Verfahren und Vorrichtungen bekannt. In der EP 0 063 644 wird zum Beispiel ein Verfahren beschrieben, bei dem nach einer Vorwahl bestimmter Randbedingungen wie relativer Härtewert, Aufnahmefeldgröße, Film- und Folienempfindlichkeit usw. zunächst die Röntgenaufnahme bis zum Erreichen einer programmierten Dosis mit einer programmierten Röhrenspannung und einem programmierten Röhrenstrom geschaltet und die bis dahin verstrichene Zeit gemessen wird. Die Röntgenaufnahme wird dann mit einem dieser gemessenen Zeit zugeordneten und gespeicherten Wert für die Röhrenspannung und das mAs-Produkt fortgesetzt. Auf diese Weise wird die Dosis und /oder die Dosisleistung der Röntgenstrahlung an die Dichte des abzubildenden Objektes (Objekttransparenz) angepasst.

Nachteile dieser und anderer, teil-automatisierter Verfahren und Vorrichtungen, mit denen einer der genannten Parameter in Abhängigkeit von den anderen, voreingestellten oder gemessenen Parametern ermittelt wird, bestehen darin, dass die Bildqualität in wesentlichem Maße von der Fähigkeit der Bedienperson abhängig ist, die entsprechende Vorwahl in geeigneter Weise zu treffen. Auch bei dynamischen Prozessen, bei denen zum. Beispiel Aufnahmen mit einem bewegten Kontrastmittel gemacht werden, stoßen diese Verfahren und Vorrichtungen häufig an ihre Grenze, da insbesondere in dem Fall, in dem die Dosisleistung nicht optimal eingestellt wird, eine durch eine Absorptionsänderung automatisch geregelte Verlängerung der Belichtungszeit zu Unschärfen in dem Bild führen kann.

Eine Aufgabe, die der Erfindung zugrunde liegt, besteht deshalb darin, ein Verfahren zur Belichtung von Röntgenaufnahmen zu schaffen, mit dem insbesondere bei dynamischen Untersuchungen der eingangs genannten Art eine weiter verbesserte Bildqualität erzielt werden kann.

Weiterhin soll ein Röntgengenerator mit Belichtungsautomatik zur Durchführung dieses Verfahrens geschaffen werden, bei dem der Grad der Automatisierung weiter erhöht und somit die Bildqualität nicht mehr in so starkem Maße von der Erfahrung einer Bedienperson abhängig ist.

Gelöst wird diese Aufgabe gemäß Anspruch 1 mit einem Verfahren der genannten Art, das sich durch folgende Verfahrensschritte auszeichnet: Voreinstellen einer maximalen Belichtungszeit (Tmax) für eine Aufnahme, die im wesentlichen nicht überschritten werden soll; Voreinstellen einer Aufnahme-kV-Startspannung für eine Röntgenröhre in Abhängigkeit von einem zu untersuchenden Objekt; Beginnen der Röntgenaufnahme und Erfassen einer Röntgenstrahlen-Absorption des Objektes und entweder Regeln der Belichtung der Aufnahme durch Verändern der Aufnahme-kV-Startspannung mit der maximalen Belichtungszeit (Tmax), wenn die Röntgenstrahlen-Absorption größer oder gleich einem ersten Schwellwert (B) ist; oder Regeln der Belichtung der Aufnahme durch Verändern der Belichtungszeit bei konstanter Aufnahme-kV-Startspannung, wenn die Röntgenstrahlen-Absorption kleiner als der erste Schwellwert (B) ist.

Unter "Voreinstellen" einer maximalen Belichtungszeit Tmax sowie einer Aufnahme-kV-Startspannung soll dabei sowohl eine Voreinstellung durch eine Bedienperson, als auch eine zum Beispiel durch eine Mikroprozessoreinheit in Abhängigkeit von anderen Eingaben vorgenommene Voreinstellung oder eine feste Programmierung der beiden Größen verstanden werden. Dies betrifft auch alle weiteren, im folgenden erläuterten Einstellmöglichkeiten.

Die Aufgabe wird ferner gemäß Anspruch 3 mit einem Röntgengenerator mit Belichtungautomatik die so geschaltet ist, dass sie im Betrieb das Verfahren durchführt, der sich dadurch auszeichnet, dass die Belichtungsautomatik einen Mehrfachregler zur Ansteuerung einer Röntgenröhre mit einem Dosisregler und mindestens einem Dosisleistungsregler aufweist, die durch einen Dosisleistungssensor zum Erfassen einer Röntgenstrahlen-Absorption des Objektes beaufschlagbar sind.

Ein besonderer Vorteil dieser Lösungen besteht darin, dass insbesondere bei dynamischen Untersuchungen, bei denen sich die Absorption des Untersuchungsobjektes ändert, die Gefahr von unscharfen Bildern aufgrund einer zu langen Belichtungszeit vermieden wird. Außerdem kann die Regelung der Aufnahme-kV-Startspannung·(im allgemeinen die Organ-kV-Spannung eines zu untersuchenden Organs) bei Einsatz einer entsprechend schnellen Schaltungstechnik bereits nach etwa 1 bis 2 ms abgeschlossen sein, so dass die Aufnahme dann mit im wesentlichen konstanter Aufnahme-kV-Spannung belichtet wird, wenn die Absorption während der Aufnahme konstant bleibt.

Die Unteransprüche haben vorteilhafte und Weiterbildungen der Erfindung zum Inhalt.

Mit den Ausführungen gemäß Anspruch 2 kann das Verfahren durch Arbeitsbereiche noch besser auf bestimmte Untersuchungsbedingungen bzw. Objekteigenschaften abgestimmt werden.

Mit der Ausführung gemäß Anspruch 4 ist eine sehr schnelle Regelung der Dosisleistung in beiden Richtungen möglich.

Mit den Ausführungen gemäß den Ansprüchen 5 und 6 kann eine maximale bzw. eine minimale Belichtungszeit für eine Aufnahme eingestellt werden. Mit der Ausführung gemäß Anspruch 7 ist ein Sollwert der Dosis bzw. Dosisleistung einstellbar, während mit der Ausführung gemäß Anspruch 8 ein Startwert einer Aufnahme-kV-Spannung deren Regelbereich sowie deren Maximalwert einstellbar sind .

Weitere Einzelheiten, Merkmale und Vorteile der Erfindung ergeben sich aus der folgen den Beschreibung einer bevorzugten Ausführungsform anhand der Zeichnung Es zeigt:
- Fig. 1: ein Prinzipschaltbild eines Röntgensystems mit einem erfindungsgemäßen Röntgengenerator; und
- Fig 2: ein Diagramm des zeitlichen Verlaufes der Belichtungszeit in Abhängigkeit von einer Objektdichte.

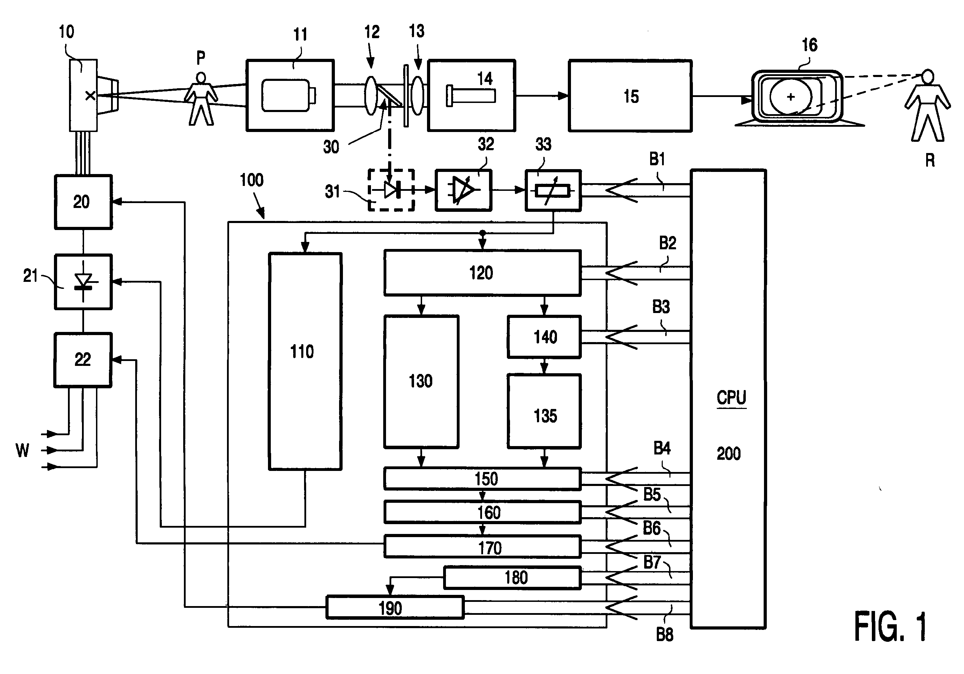

Zu den wesentlichen Komponenten eines Röntgensystems gehören gemäß Figur 1 eine Röntgenröhre 10 zur Erzeugung von Röntgenstrahlen, die einen Patienten P durchsetzen und ein Abbild des zu untersuchenden Bereiches auf einen Bildverstärker 11 projizieren. Dieses Abbild wird in bekannter Weise verstärkt und in Lichtsignale umgewandelt, die dann von einer Linsen- und Blendenanordnung 12, 13 gebündelt und von einer Kamera 14 aufgenommen und in entsprechende elektrische Signale umgewandelt werden. Diese Signale werden dann einer im allgemeinen digitalen Bildverarbeitungseinrichtung 15 zugeführt, an die ein Monitor 16 zur Betrachtung des zu untersuchenden Bereiches des Patienten P durch einen Radiologen R angeschlossen ist.

Die Röntgenröhre 10 wird durch einen Hochspannungsgenerator 20 gespeist. Der Hochspannungsgenerator 20 ist über einen Leistungsschalter 21 zum Ein-bzw. Ausschalten der Hochspannung mit einem Konverter 22 verbunden, der zur Umwandlung einer allgemeinen Netzspannung W in eine geeignete Eingangsspannung für den Hochspannungsgenerator 20 dient und damit den Aufnahme-kV-Spannungswert (das ist die an der Röntgenröhre anliegende Hochspannung) bestimmt.

Die drei eingangs genannten Parameter werden mit diesen Komponenten wie folgt beeinflusst bzw. verändert:

Die Belichtungszeit (Aufnahmezeit) und die Strahlendosis, der der Patient ausgesetzt wird, ist durch entsprechendes Ansteuern des Leistungsschalters 21 mit einem ersten Steuersignal einstellbar. Die Dosisleistung der Röntgenröhre 10 wird durch Ansteuern des Konverters 22 und somit durch Verändern der Aufnahme-kV-Spannung mit einem zweiten Steuersignal eingestellt. Darüber hinaus kann der Heizstrom der Röntgenröhre 10 mit einem dritten Steuersignal eingestellt werden, das an einem entsprechenden Eingang des Hochspannungsgenerators 20 anliegt. Diese drei Steuersignale werden mit einem Mehrfachregler 100 erzeugt, der mit einer Mikroprozessoreinheit 200 über eine Mehrzahl von Busleitungen B2 bis B8 gesteuert wird.

Zur Erzeugung einer Regelgröße für den Mehrfachregler 100 in Form einer im Bereich der Kamera 14 auftreffenden Dosis bzw. Dosisleistung, die durch die Röntgenstrahlenabsorption des Patienten und andere bildbeeinflussende Objekte bestimmt wird, ist schließlich an der Kamera 14 ein Strahlteiler 30 angeordnet, mit dem aus der Röntgenstrahlung ein Teilstrahl ausgeblendet und zur Erzeugung eines Dosis- bzw. Dosisleistungssignals auf einen entsprechenden Sensor 31 (Fotosensor) gerichtet wird. Der Fotosensor 31 ist mit einem Kalibrator 32 verbunden, mit dem eine auf diese Dosis bzw. Dosisleistung normierte Spannung erzeugt wird. Diese Spannung liegt an einem Teiler 33 an, mit dem ein Sollwert der Dosis (zum Beispiel 0,66 µGy) bzw. der Dosisleistung (zum Beispiel 66 µGy/s) eingestellt werden kann. Der Teiler ist zu diesem Zweck über einen ersten Bus B1 mit der Mikroprozessoreinheit 200 verbunden ist. Das Ausgangssignal des Teilers 33 liegt an dem Mehrfachregler 100 an.

Der Mehrfachregler 100 beinhaltet einen an sich bekannten Dosisregler 110 (Amplimat), dem das Ausgangssignal des Teilers 33 zugeführt wird, und der einen Integrator für dieses Signal sowie einen Komparator aufweist. Der Dosisregler erzeugt das erste Steuersignal für den Leistungsschalter 21 und regelt die Belichtungszeit für die Aufnahme in Abhängigkeit von der an dem Fotosensor 31 gemessenen Dosis und dem über den Teiler 33 eingestellten Dosis-Sollwert.

Weiterhin ist ein Nominalzeitwähler 120 für einen Bereich von zum Beispiel etwa 4 bis 4000 ms vorsehen, der ebenfalls das Ausgangssignal des Teilers 33 erhält und der durch die Mikroprozessoreinheit 200 über den zweiten Bus B2 zur Einstellung eines Sollwertes einer oberen Grenze Tmax eines Belichhtungszeitfensters oder einer maximalen Belichtungszeit (zum Beispiel 50 ms) angesteuert werden kann.

Der Nominalzeitwähler 120 ist über einen ersten Ausgang mit einem ersten Dosisleistungsregler 130 und über einen zweiten Ausgang mit einer Einheit 140 zur Erzeugung eines Zeitfenster-Faktors von zum Beispiel zwischen 1 und 10 (realisiert als Abschwächer mit einem Faktor zwischen 1 und 0,1) verbunden, mit dem aus der gewählten maximalen Belichtungszeit Tmax ein Sollwert einer unteren Grenze Tmin des Belichtungszeitfensters oder einer minimalen Belichtungszeit (zum Beispiel 10 ms) erzeugt wird Der Faktor wird entsprechend der über die Mikroprozessoreinheit 200 und den dritten Bus B3 eingegebenen minimalen Belichtungszeit Tmin bestimmt. Der Ausgang der Einheit 140 liegt an dem Eingang eines zweiten Dosisleistungsreglers 135 an.

Der erste Dosisleistungsregler 130 beinhaltet im wesentlichen einen PID-Regler mit mittlerer Geschwindigkeit im Bereich von etwa 5 kHz und dient nur für positive Korrekturen, das heißt für eine Aufwärts-Regelung (Erhöhung) der Aufnahme-kV-Spannung für die Röntgenröhre. Der zweite Dosisleistungsregler 135 beinhaltet im wesentlichen einen PID-Regler mit hoher Geschwindigkeit im Bereich von etwa 10 kHz und dient ausschließlich für negative Korrekturen, das heißt für eine Abwärts-Regelung (Verminderung) der Aufnahme-kV-Spannung.

Die Ausgänge der beiden Dosisleistungsregler 130,135 werden einem ersten Begrenzer 150 zugeführt, wobei der Begrenzer durch die Mikroprozessoreinheit 200 über den vierten Bus B4 angesteuert wird und zur Einstellung von Grenzwerten dient, bis zu denen die Aufnahme-kV-Spannung mit den Dosisleistungsreglern maximal erhöht bzw. vermindert werden darf (zum Beispiel um +25 kV oder +15 kV bzw. um -15 kV oder -10 kV gegenüber einem Startwert).

Der Sollwert einer Aufnahme-kV-Startspannung ist über die Mikroprozessoreinheit und den fünften Bus B5 einstellbar. Bei der Untersuchung von Menschen ist dieser Sollwert im allgemeinen die Organ-kV-Spannung des zu untersuchenden Organs (zum Beispiel 70 kV). Der fünfte Bus B5 ist zu diesem Zweck an einen Signalmischer 160 geführt, der an den Ausgang des ersten Begrenzers 150 angeschlossen ist und zur Erzeugung der Aufnahme-kV-Spannung durch Summierung von deren eingestelltem Startwert mit den durch die Dosisleistungsregler erzeugten Spannungswerten dient.

Weiterhin ist ein zweiter Begrenzer 170 für die durch den Signalmischer 160 summierte Aufnahme-kV-Spannung vorgesehen, mit dem über die Mikroprozessoreinheit 200 und den sechsten Bus B6 ein zulässiger Gesamtbereich dieser Spannung von zum Beispiel zwischen 55 und 125 kV eingestellt werden kann. Der zweite Begrenzer 170 erzeugt an seinem Ausgang das zweite Steuersignal, das schließlich an den Konverter 22 geführt ist, um über diesen die allgemeine Netzspannung W so umzusetzen, dass der entsprechende Aufnahme-kV-Spannungswert durch den Hochspannungsgenerator 20 erzeugt werden kann.

Der Mehrfachregler 100 umfasst weiterhin eine Einheit 180 zur Erzeugung eines Röhrenstromfaktors in Abhängigkeit von einem gewählten Bildverstärkerformat, wobei ein wünschter Stromfaktor mittels der Mikroprozessoreinheit 200 über den siebten Bus B7 eingegeben werden kann und zum Beispiel zwischen 1 und 2,5 liegt.

Der Ausgang der Einheit 180 ist schließlich mit einer Einheit 190 zur Erzeugung eines Sollwertes des Heizstroms in Abhängigkeit von einem über die Mikroprozessoreinheit und den achten Bus B8 einstellbaren Basiswert (zum Beispiel 200 mA) sowie dem durch die Einheit 180 ermittelten Röhrenstromfaktor verbunden. Der Ausgang der Einheit 190, die das dritte Steuersignal erzeugt, ist an den Hochspannungsgenerator 20 geführt und steuert diesen so an, dass der ermittelte Sollwert des Heizstroms durch die Röntgenröhre 10 fließt.

Die vorteilhaften Eigenschaften werden besonders bei der Anwendung des Röntgensystems für dynamische Untersuchungen mit schnellen Absorptionsänderungen (zum Beispiel Colonuntersuchungen mit einem Kontrastmittel) deutlich. Hierzu sei auf folgendes hingewiesen: da auf Grund von gesetzlichen Bestimmungen die zulässige Strahlendosis an dem Bildempfänger im allgemeinen festgelegt ist, wird bei bekannten Belichtungssteurungen diese Dosis im wesentlichen konstant gehalten. Dies hat zur Folge, dass bei einer Absorptionsänderung in dem zu untersuchenden Objekt während der Aufnahme mehrerer Bilder infolge des sich bewegenden Kontrastmittels die Belichtungszeit entsprechend verändert wird. Dabei besteht die Gefahr, dass eine zur Erzielung einer schatten Aufnahme maximale Belichtungszeit von etwa 100 ms deutlich überschritten wird Erfasst man die Patientendicken in Form von Wassergleichwerten, so ergibt sich für einen Bereich von 120 bis 450 mm Wassergleichwerten ein durch die bekannten Belichtungsautomaten eingestellter Belichtungszeitbereich zwischen etwa 3,3 und 530 ms und somit eine Änderung mit einem Faktor von etwa 160. Dies wird als viel zu hoch angesehen und kann zu unscharfen Bildern führen.

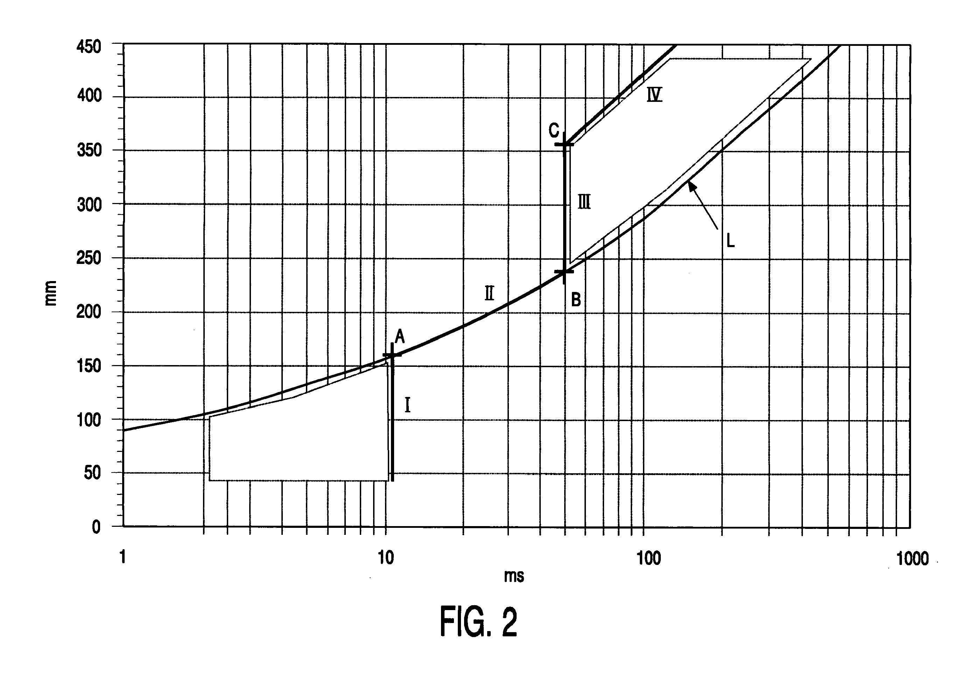

Im folgenden soll nun die Funktionsweise des erfindungsgemäßen Röntgengenerators mit dem Mehrfachregler (Mehrstufen- oder Mehrbereichsregler) anhand der Figur 2 erläutert werden.

Bei dem erfindungsgemäßen Mehrfachregler 100 werden mit den Dosisleistungsreglern 130, 135 die Aufnahme-kV-Werte in Abhängigkeit von der mit dem Sensor 31 erfassten Dosisleistung, die im wesentlichen die Objektdichte (d h. die Patientendicke) darstellt, so geregelt, dass ein einstellbarer Belichtungszeitbereich nicht verlassen und insbesondere die maximale Belichtungszeit Tmax zur Sicherstellung von scharfen Aufnahmen nicht bzw. erst bei wesentlich höheren Absorptionswerten überschritten wird, als dies bei bekannten Belichtungsautomaten der Fall ist. Der Dosisregler 110 regelt parallel dazu in Abhängigkeit von der mit dem Sensor 31 erfassten Dosis in bekannter Weise die Dosis pro Aufnahme auf einen für eine ausreichende Belichtung erforderlichen (im wesentlichen konstanten) Wert, indem die Röntgenröhre nach Ablauf der dafür benötigten Belichtungszeit (bzw. Tmax) abgeschaltet wird Die dafür wiederum erforderliche Dosisleistung D_{R} wird nach der Formel D_{R} = D /t bestimmt.

Zur Verdeutlichung dieser Funktionsweise ist in Figur 2 der Zusammenhang zwischen der Belichtungszeit (horizontale Achse) und der durch den Wassergleichwert (vertikale Achse) ausgedrückten Objektdichte (Röntgenstrahlenabsorption) dargestellt, wobei für die folgende Erläuterung weitere, die Absorption erhöhende Bildbeeinflussungselemente der Objektdichte zugerechnet werden sollen.

Der Regler arbeitet gemäß dieser Darstellung in vier verschiedenen Arbeitsbereichen. In einem ersten Bereich I, der für relativ dünne Patienten vorgesehen ist und sich unterhalb und bis zu einem Wassergleichwert von etwa 160 mm (Punkt A) erstreckt, wird die Belichtungszeit auf dem minimalen Wert Tmin konstant gehalten, der durch schaltungstechnische Gegebenheiten (zum Beispiel parasitäre Kapazitäten) bestimmt ist und in dieser Darstellung etwa 11 ms beträgt, oder über den dritten Bus B3 eingestellt werden kann. In diesem Bereich wird die Belichtung der Aufnahme mit dem zweiten Dosisleistungsregler 135 durch gleitende Veränderung der Aufnahme-kV-Spannung angepasst, die ausgehend von dem über den fünften Bus B5 voreingestellten Startwert (mittlere Organ-kV-Spannung) in Abhängigkeit von der Objektdichte um bis zu maximal etwa -15 kV reduziert wird, so dass das erzeugte Bild mit der minimalen Belichtungszeit Tmin nicht überbelichtet wird.

In einem zweiten Bereich II, der für Objektdichten mit Wassergleichwerten von etwa 160 bis etwa 240 mm (zwischen den Punkten A und B) vorgesehen ist, bleibt die Aufnahme-kV-Spannung konstant auf ihrem voreingestellten Startwert oder mittleren Organ-kV-Wert (bei dem anhand von Figur 1 erläuterten Beispiel bei etwa 70 kV), und die Belichtungszeit t wird durch den Dosisregler 110 in Abhängigkeit von der Objektdichte (Wassergleichwert) zwischen etwa 11 und etwa 50 ms geregelt.

Ein sich daran anschließender dritter Bereich III ist für Objektdichten mit Wassergleichwerten von etwa 240 bis etwa 360 mm (zwischen den Punkten B und C) vorgesehen. In diesem Bereich bleibt die Belichtungszeit t konstant bei dem voreingestellten maximalen Wert Tmax (hier etwa 50 ms), und die Aufnahme-kV-Spannung wird ausgehend von dem voreingestellten Startwert mit dem ersten Dosisleistungsregler 130 gleitend in Abhängig keit von der Objektdichte (Wassergleichwert) um bis zu maximal etwa 25 kV erhöht, so dass mit der maximalen Belichtungszeit Tmax eine ausreichende Belichtung erzielt wird (D_{R} = D /Tmax).

Ein vierter Bereich IV (oberhalb des Punktes C) ist schließlich für Objektdichten mit Wassergleichwerten von etwa 360 bis etwa 450 mm vorgesehen. In diesem Bereich bleibt die Aufnahme-kV-Spannung konstant auf ihrem maximalen, durch den zweiten Begrenzer 170 bestimmten Wert, der sich aus der Summe aus dem voreingestellten Startwert und der maximalen Erhöhung von etwa 25 kV ergibt. Die Belichtung wird durch den Dosisregler 110 durch gleitende Verlängerung der Aufnahmezeit t von etwa 50 ms bis auf maximal etwa 150 ms erhöht. Auch wenn diese maximale Aufnahmezeit bei diesem Beispiel höher ist, als der zuvor genannte maximale Wert für scharfe Aufnahmen (etwa 100ms), so ist dieser Wert, zumal er sich nur für besonders dicke Patienten ergibt, als Kompromiss im Hinblick auf die Objektdichte und die Bewegungsunschärfe medizinisch noch vertretbar.

Zu Beginn jeder Bildaufnahme wird in Abhängigkeit von der dem Mehrfachregler 100 zugeführten Regelgröße, d. h der mit dem Sensor 31, dem Kalibrator 32 und dem Teiler 33 erzeugten Spannung (Regelspannung), sowie der eingestellten maximalen und minimalen Belichtungszeit Tmax, Tmin, einer der Dosisleistungsregler (sofern erforderlich) aktiviert.

Zu diesem Zweck dienen der Nominalzeitwähler 120 und die Zeitfensterfaktor-Einheit 140. Weiterhin ist den beiden Dosisteistungsreglern 130, 135 jeweils eine Sollwertreferenz von zum Beispiel 1,0 Volt zugeordnet, wobei der zweite Dosisleistungsregler 135 aufgrund des durch die Zeitfensterfaktor-Einheit 140 realisierten Abschwächungsfaktors eine wirksame Sollwertreferenz von zum Beispiel 5,0 Volt (gebildet durch die Formel: Sollwertreferenz /Abschwächungsfaktor = wirksame Sollwertreferenz) aufweist.

In dem Nominalzeitwähler 120 wird die Regelspannung in Abhängigkeit von der über den zweiten Bus B2 vorgenommenen Einstellung der maximalen Belichtungszeit Tmax gewichtet, d h mit einem entsprechenden Faktor beaufschlagt.

Wenn diese gewichtete Regelspannung bei Beginn der Aufnahme kleiner oder gleich der ersten Sollwertreferenz ist (entsprechend einer hohen Absorption und /oder niedrigen Tmax), aktiviert sich der erste Dosisleistungsregler 130 und erhöht die Aufnahme-kV-Spannung in der beschriebenen Weise (Bereich III).

Weiterhin wird in der Einheit 140 die von dem Nominalzeitwähler 120 zugeführte gewichtete Regelspannung in Abhängigkeit von der über den dritten Bus B3 vorgenommenen Einstellung der minimalen Belichtungszeit Tmin mit einem entsprechenden Zeitfensterfaktor beaufschlagt.

Wenn diese gewichtete und mit dem Zeitfensterfaktor beaufschlagte Regelspannung größer oder gleich der zweiten Sollwertreferenz ist (niedrige Absorption und /oder große Tmin), aktiviert sich der zweite Dosisleistungsregler 135 und vermindert die Aufnahme-kV-Spannung in der beschriebenen Weise (Bereich I).

Wenn die Regelspannungen zwischen den Sollwertreferenzen liegen, bleibt die Aufnahme-kV-Spannung unverändert auf ihrem Startwert, und die Belichtungsregelung wird ausschließlich durch den Dosisregler 110 vorgenommen (Bereich II).

Für den Bereich der Wassergleichwerte von 120 bis 450 mm ergibt sich somit mit dem erfindungsgemäßen Mehrfachregler ein Aufnahmezeitbereich von 11 bis 150 ms, was einer Änderung mit einem Faktor von nur 13,6 entspricht. Weiterhin zeigt sich, dass der zum Beispiel für Colonuntersuchungen geforderte Wert für die maximale Aufnahmezeit von 100 ms erst bei einem Wassergleichwert von etwa 420 mm überschritten wird, während dies ohne Anwendung des erfindungsgemäßen Mehrfachreglers (oder bei einer Regelung nur durch den Dosisregler 110) entsprechend der in Figur 2 ebenfalls eingezeichneten (dünnen) Linie L schon bei einem Wassergleichwert von etwa 290 mm der Fall wäre.

Wenn für Tmax und Tmin gleiche Werte gewählt werden, bedeutet dies, dass die Belichtung bei diesem konstanten Wert ausschließlich über eine Veränderung der Aufnahme-kV-Spannung geregelt wird.

Vorzugsweise sind über die Einstellung von Tmax und Tmin drei verschiedene Bereiche II wählbar, die zum Beispiel zwischen 5 und 25 ms (für Kinder) und zwischen 10 und 50 ms (wie dargestellt) sowie zwischen 20 und 100 ms (für Erwachsene) liegen.

Unabhängig von diesen Bereichen wird über die Mikroprozessoreinheit und den ersten Bus B1 die Dosis eingestellt, die in Abhängigkeit von der gewünschten Bildqualität (Signal-Rausch-Verhältnis), gesetzlichen Bestimmungen, der zumutbaren Belastung des Patienten und /oder dem Bildverstärker-Format durch den Anwender gewählt wird. Bei einer Umschaltung der Bildverstärkerformate wird der Dosisfaktor auf den Röhrenstrom (Aufnahmestrom) übertragen, so dass die gewählte Aufnahme-kV-Spannung erhalten bleibt. Bei Bildverstärker-Formaten von zum Beispiel 38 /25 /17 cm wird der Röhre-Strom zum Beispiel um die Faktoren 1 /1,6 /2,5 erhöht. Hierzu dienen die oben erläuterten Einheiten 180 und 190.

Die Regelung der Aufnahme-kV-Spannung in den Bereichen I und III zwischen einem minimalen und einem maximalen Wert ist über die Mikroprozessoreinheit 200 und den vierten Bus B4 einstellbar. Vorzugsweise sind zwei Spannungbereiche vorgesehen, die sich zum Beispiel zwischen -15 und +25 kV (wie dargestellt) oder zwischen -10 und +15 kV gegenüber der Aufnahme-kV-Startspannung (mittlere Organ-kV-Spannung) erstrecken.

Für dieses Beispiel ist der erste Dosisleistungsregler 130 (Positivregler) zur Erhöhung der Aufnahme-kV-Startspannung im Bereich III um maximal +15 oder +25 kV vorgesehen, während der zweite Dosisleistungsregler 135 (Negativregler) zur Verminderung der Aufnahme-kV-Startspannung im Bereich I um maximal -10 bzw. -15 kV dient. Dadurch, dass für jede Richtung ein gesonderter Regler verwendet wird, sowie durch geeignete Auswahl einer möglichst schnellen (insbesondere analogen) Schaltungstechnik, ist eine entsprechend schnelle Erhöhung und Verminderung der Aufnahme-kV-Spannung (und damit der Röntgenstrahlen-Dosisleistung) möglich, so dass die Regelung der Aufnahme-kV-Spannung etwa 1 bis 2 ms nach Beginn der Aufnahme beendet ist und dann die Aufnahme-kV-Spannung bis zur Beendigung der Aufnahme durch den Dosisregler 110 im wesentlichen konstant bleibt.

Zusammengefasst wird also mit dem erfindungsgemäßen Mehrfachregler die Dosisleistung D_{R} der Röntgenröhre in Abhängigkeit von der Objektdichte (Patientendicke) so geregelt, dass der Dosisregler ein durch den Bereich II definiertes Zeitfenster nicht bzw. erst bei einer wesentlich höheren Objektdichte (Punkt C) verlassen muss, so dass eine optimale Bildschärfe auch bei dynamischen Aufnahmen sichergestellt ist. Die Belichtungszeit t wird dabei unabhängig und rückwirkungsfrei von der durch den Anwender eingestellten Dosis D nach der Formel t = D /D_{R} geregelt (Bereiche II und IV). In den Bereichen I und III, in denen die Belichtungszeit auf Tmin bzw. Tmax konstant ist, wird innerhalb von 1 bis 2 ms nach Aufnahmebeginn die Aufnahme-kV-Spannung nach der Formel D_{R}=D /t eingestellt und bleibt dann bis zum Ende der Aufnahme (Abschalten der Belichtung durch den Dosisregler 110) im wesentlichen konstant.

Zur Klarstellung sei an dieser Stelle angemerkt, dass die erfasste Röntgenstrahlen-Absorption "des Objektes" stets auch die Absorption durch andere Elemente beinhaltet, die sich in dem Strahlengang zwischen der Röntgenröhre und dem Sensor 31 befinden, der unmittelbar an der Aufnahmeeinrichtung (Kamera 14) angeordnet ist. Auf diese Weise wird sichergestellt, dass die Belichtung unter Berücksichtigung des Untersuchungsobjektes und aller anderen bildbeeinflussenden Elemente (wie Bildverstärker 11, Linse 12 usw.) optimal erfolgt.

Das beschriebene Regelkonzept ist insbesondere bei der schnellen Aufnahme einer Vielzahl von aufeinanderfolgenden Bildern vorteilhaft, da in diesem Fall durch Einstellung einer entsprechend geringen maximalen Belichtungszeit Tmax ("Zeitpriorität") verhindert werden kann, dass die Belichtungszeit länger Wird als die für ein einzelnes Bild zur Verfügung stehende Zeit.

Ein weiterer wesentlicher Vorteil des Mehrfachreglers besteht darin, dass er sehr universell einsetzbar ist. Dies beruht im wesentlichen darauf, dass alle relevanten Daten für die Aufnahme, wie zum Beispiel die Aufnahme-kV-Startspannung, die in Abhängigkeit von dem zu untersuchenden Organ gewählt wird, das Belichtungszeit-Fenster, die Regelungsbereiche der Aufnahme-kV-Spannung usw. über eine Mikroprozessoreinheit gesteuert werden.

Der Regler ist weiterhin unabhängig von dem gewählten Bildempfänger einsetzbar und kann sowohl mit den bekannten Bildverstärker-Videokameras, als auch mit den neuen digitalen Flachdetektoren (FDXD) oder Speicherfolien (PCR) verwendet werden. Das Dosisleistungssignal wird dabei im erstgenannten Fall (indirekte Technik) mit einem Fotosensor mit schneller Signalreaktionszeit (< 100 µs) und bei den direkten Techniken (FDXD, PCR) mit einer Ionisationskammer mit Ausgang für das Dosisleistungssignal mit schneller Signaleaktionszeit (< 100 µs) gewonnen, wobei vorzugsweise eine Kalibrierung auf absolute Dosiswerte (µGy) beziehungsweise absolute Dosisleistungswerte (µGy/s) bei einer Referenzspannung von 1,0 Volt vorgenommen wird

Die Steuerung der Reglerstrategie kann in eine EPX-Datenbankstruktur eingebunden werden.

Der Mehrfachregler kann sowohl zur Belichtung einer einzelnen Aufnahme, als auch zur Belichtung der Aufnahmen einer Bildsequenz (Bewegtbilder), während der sich die Absorption des Objektes verändert, verwendet werden.

Der Mehrfachregler kann darüber hinaus für alle medizinischen Techniken, wie zum Beispiel auch Tomographie, digitale Sofortbildtechnik (DSI), digitale Aufnahmetechnik we Kinotechnik sowie konventionelle Film-Folientechnik usw. sowie für andere Röntgensysteme eingesetzt werden.

## Patentansprüche

1. Verfahren zur Belichtung von Röntgenaufnahmen, insbesondere von Aufnahmen bei dynamischen Röntgenuntersuchungen eines Objektes,
**gekennzeichnet durch** folgende Verfahrensschritte:
Voreinstellen einer maximalen Belichtungszeit (Tmax) für eine Aufnahme, die nicht überschritten werden soll;
Voreinstellen einer Aufnahme-kV-Startspannung für eine Röntgenröhre in Abhängigkeit des zu untersuchenden Objekt;
Beginnen der Röntgenaufnahme und Erfassen einer Röntgenstrahlen-Absorption des Objektes; und entweder
- Regeln der Belichtung der Aufnahme **durch** Verändern der Aufnahme-kV-Startspannung unter Verwendung der maximalen Belichtungszeit (Tmax), wenn die Röntgenstrahlen-Absorption größer oder gleich einem ersten Schwellwert (B) ist; oder
- Regeln der Belichtung der Aufnahme **durch** Verändern der Belichtungszeit bei konstanter Aufnahme-kV-Startspannung, wenn die Röntgenstrahlen-Absorption kleiner als der erste Schwellwert (B) ist.

2. Verfahren nach Anspruch 1,
**dadurch gekennzeichnet,**
**dass** eine minimale Belichtungszeit (Tmin) für eine Aufnahme voreingestellt wird, wobei die Belichtung der Aufnahme entweder durch Verändern der Aufnahme-kV-Startspannung unter Verwendung der minimalen Belichtungszeit (Tmin) erfolgt, wenn die Röntgenstrahlen-Absorption kleiner oder gleich einem zweiten Schwellwert (A) ist; oder die Belichtung der Aufnahme durch Verändern der Belichtungszeit bei konstanter Aufnahme-kV-Startspannung erfolgt, wenn die Röntgenstrahlen-Absorption größer als der zweite Schwellwert (A) ist.
**dadurch gekennzeichnet**,

3. Röntgengenerator mit Belichtungsautomatik die so geschaltet ist, dass sie im Betrieb das Verfahren nach Anspruch 1, 2 oder 3 durchführt,
**dadurch gekennzeichnet,**
**dass** die Belichtungsautomatik einen Mehrfachregler (100) zur Ansteuerung einer Röntgenröhre mit einem Dosisregler (110) und mindestens einem Dosisleistungsregler (130, 135) aufweist, die durch einen Dosisleistungssensor (31) zum Erfassen einer Röntgenstrahlen-Absorption des Objektes beaufschlagbar sind.

4. Röntgengenerator nach Anspruch 3,
**dadurch gekennzeichnet,**
**dass** ein erster Dosisleistungsregler (130) zur Erhöhung der Dosisleistung der Röntgenröhre sowie ein zweiter Dosisleistungsregler (135) zur Verminderung der Dosisleistung der Röntgenröhre vorgesehen ist, die parallel geschaltet sind.

5. Röntgengenerator nach Anspruch 4,
**gekennzeichnet durch** eine erste Einheit (120), mit der die maximale Belichtungszeit (Tmax) für eine Aufnahme voreinstellbar ist und die zwischen den Dosisleistungssensor (31) und die beiden Dosisleistungsregler (130, 135) geschaltet ist und diese beaufschlagt.

6. Röntgengenerator nach Anspruch 5,
**gekennzeichnet durch** eine zweite Einheit (140), mit der eine minimale Belichtungszeit (Tmin) für eine Aufnahme voreinstellbar ist und die zwischen die erste Einheit (120) und den zweiten Dosisleistungsregler (135) geschaltet ist und diesen beaufschlagt.

7. Röntgengenerator nach Anspruch 3,
**dadurch gekennzeichnet,**
**dass** der Dosisleistungssensor (31) über einen Kalibrator (32) und einen Teiler (33) zum Einstellen eines Sollwertes der Dosis bzw. Dosisleistung mit dem Dosisregler (110) und dem mindestens einen Dosisleistungsregler (130, 135) verbunden ist.

8. Röntgengenerator nach Anspruch 3,
**gekennzeichnet durch** eine dritte Einheit (150, 160, 170), die **durch** den mindestens einen Dosisleistungsregler (130, 135) beaufschlagt wird, zur Einstellung eines Startwertes einer Aufnahme-kV-Spannung für eine Röntgenröhre sowie von deren Regelbereich und deren Maximalwert.

9. Röntgengenerator nach Anspruch 3,
**gekennzeichnet durch** eine vierte Einheit (180, 190) zur Erzeugung eines Heizstroms für die Röntgenröhre in Abhängigkeit von einem Basiswert sowie einem in Abhängigkeit von einem Bildverstärkerformat einstellbaren Röhrenstromfaktor.

10. Röntgengenerator nach Anspruch 9,
**gekennzeichnet durch** eine Mikroprozessoreinheit (200), mit der der Teiler (33) sowie die erste bis vierte Einheit (120; 140; 150, 160, 170; 180; 190) über eine Mehrzahl von Busleitungen (B 1 bis B8) einstellbar ist.

11. Röntgensystem mit mindestens einer Einrichtung zur Bilderzeugung und Bildverarbeitung,
**gekennzeichnet durch** einen Röntgengenerator nach einem der Ansprüche 3 bis 10.

## Claims

1. A method for X-ray exposure control, notably for exposures carried out during dynamic X-ray examinations of an object, **characterized in that** the method includes the following steps:
presetting for an exposure a maximum exposure time (Tmax) which may not be exceeded;
presetting an exposure kV start voltage for an X-ray tube in dependence on the object to be examined;
starting the X-ray exposure and measuring an X-ray absorption of the object; and either
controlling the exposure by changing the exposure kV start voltage at the maximum exposure time (Tmax) when the X-ray absorption is higher than or equal to a first threshold value (B), or
controlling the exposure by changing the exposure time at a constant exposure kV start voltage when the X-ray absorption is less than the first threshold value (B).

2. A method as claimed in claim 1, **characterized in that** a minimum exposure time (Tmin) is preset for an exposure, the exposure taking place by changing the exposure kV start voltage at the minimum exposure time (Tmin) when the X-ray absorption is less than or equal to a second threshold value (A), or the exposure taking place by changing the exposure time at a constant exposure kV start voltage when the X-ray absorption exceeds the second threshold value (A).

3. An X-ray generator comprising an automatic exposure control unit which is connected in such a way that, in operation, the control unit carries out the method as claimed in claim 1 or 2, which generator is **characterized in that** the automatic exposure control unit includes a multiple controller (100) for controlling an X-ray tube, which multiple controller includes a dose controller (110) and at least one dose rate controller (130, 135) which are subject to a dose rate sensor (31) for measuring an X-ray absorption of the object.

4. An X-ray generator as claimed in claim 3, **characterized in that** there are provided a first dose rate controller (130) for increasing the dose rate of the X-ray tube as well as a second dose rate controller (135) for reducing the dose rate of the X-ray tube, said dose rate controllers being connected in parallel.

5. An X-ray generator as claimed in claim 4, **characterized in that** it includes a first unit (120) whereby the maximum exposure time (Tmax) can be preset for an exposure and which is connected between the dose rate sensor (31) and the two dose rate controllers (130, 135) so as to act on said dose rate controllers.

6. An X-ray generator as claimed in claim 5, **characterized in that** it includes a second unit (140) whereby a minimum exposure time (Tmin) can be preset for an exposure and which is connected between the first unit (120) and the second dose rate controller (135) so as to act thereon.

7. An X-ray generator as claimed in claim 3, **characterized in that** the dose rate sensor (31) is connected, via a calibrator (32) and a divider (33) for adjusting a reference value of the dose or the dose rate, to the dose controller (110) and the at least one dose rate controller (130, 135).

8. An X-ray generator as claimed in claim 3, **characterized in that** it includes a third unit (150, 160, 170) which is subject to the at least one dose rate controller (130, 135) in order to adjust a start value of an exposure kV voltage for an X-ray tube as well as to adjust the control range and the maximum value thereof.

9. An X-ray generator as claimed in claim 3, **characterized in that** it includes a fourth unit (180, 190) for generating a filament current for the X-ray tube in dependence on a basic value as well as for forming a tube current factor which is adjustable in dependence on an image intensifier format.

10. An X-ray generator as claimed in claim 9, **characterized in that** it includes a microprocessor unit (200) whereby the divider (33) as well as the first through fourth units (120; 140; 150, 160, 170; 180, 190) can be adjusted via a plurality of bus leads (B1 to B8).

11. An X-ray system which includes at least one device for imaging and image processing, **characterized in that** it includes an X-ray generator as claimed in one of the claims 3 to 10.

## Revendications

1. Procédé destiné à l'exposition de radiographies, en particulier de clichés réalisés lors d'examens dynamiques par rayons X d'un objet,
**caractérisé par** des étapes de procédé qui suivent:
préréglage d'un temps d'exposition maximal (Tmax) pour un cliché, qui ne doit pas être dépassé;
préréglage d'une tension de démarrage en kV de cliché pour un tube à rayons X en fonction de l'objet à examiner;
commencement de la radiographie et captation d'une absorption de rayons X par l'objet; et soit
- réglage de l'exposition du cliché en modifiant la tension de démarrage en kV de cliché, avec application du temps d'exposition maximal (Tmax), lorsque l'absorption de rayons X est supérieure ou égale à une première valeur de seuil (B); soit
- réglage de l'exposition du cliché en modifiant le temps d'exposition, avec une tension de démarrage en kV de cliché constante, lorsque l'absorption de rayons X est inférieure à la première valeur de seuil (B).

2. Procédé selon la revendication 1,
**caractérisé en ce que**
un temps d'exposition minimal (Tmin) pour un cliché est préréglé, où l'exposition du cliché résulte soit d'une modification de la tension de démarrage en kV de cliché avec application du temps d'exposition minimal (Tmin) lorsque l'absorption de rayons X est inférieure ou égale à une seconde valeur de seuil (A); soit l'exposition du cliché résulte d'une modification du temps d'exposition, avec une tension de démarrage en kV de cliché constante, lorsque l'absorption de rayons X est supérieure à la seconde valeur de seuil (A).

3. Générateur de rayons X muni d'un régulateur automatique d'exposition qui est monté de façon que, en fonctionnement, il exécute le procédé selon la revendication 1, 2 ou 3,
**caractérisé en ce que**
le régulateur automatique d'exposition présente un régulateur à variables multiples (100) pour exciter un tube à rayons X avec un régulateur de dose (110) et au moins un régulateur de débit de dose (130, 135), qui peuvent être alimentés par un capteur de débit de dose (31) pour capter une absorption de rayons X par l'objet.

4. Générateur de rayons X selon la revendication 3,
**caractérisé en ce que**
sont prévus, montés en parallèle, un premier régulateur de débit de dose (130), pour augmenter le débit de dose du tube à rayons X, ainsi qu'un second régulateur de débit de dose (135), pour diminuer le débit de dose du tube à rayons X.

5. Générateur de rayons X selon la revendication 4,
**caractérisé par** une première unité (120), avec laquelle le temps d'exposition maximal (Tmax) pour un cliché est préréglable, et qui est montée entre le capteur de débit de dose (31) et les deux régulateurs de débit de dose (130, 135) et alimente ceux-ci.

6. Générateur de rayons X selon la revendication 5,
**caractérisé par** une deuxième unité (140), avec laquelle un temps d'exposition minimal (Tmin) pour un cliché est préréglable, et qui est montée entre la première unité (120) et le second régulateur de débit de dose (135) et alimente celui-ci.

7. Générateur de rayons X selon la revendication 3,
**caractérisé en ce que**
le capteur de débit de dose (31) est raccordé via un étalonneur (32) et un diviseur (33) pour régler une valeur de consigne de la dose ou bien du débit de dose avec le régulateur de dose (110) et ledit au moins un régulateur de débit de dose (130, 135).

8. Générateur de rayons X selon la revendication 3,
**caractérisé par** une troisième unité (150, 160, 170), qui est alimentée par ledit au moins un régulateur de débit de dose (130, 135), pour régler une valeur de démarrage d'une tension en kV de cliché pour un tube à rayons X, ainsi que sa plage de réglage et sa valeur maximale.

9. Générateur de rayons X selon la revendication 3,
**caractérisé par** une quatrième unité (180, 190) pour la production d'un courant de chauffage pour le tube à rayons X en fonction d'une valeur de base ainsi que d'un facteur de courant de tube réglable en fonction d'un format d'intensificateur d'image.

10. Générateur de rayons X selon la revendication 9,
**caractérisé par** une unité de microprocesseur (200), avec laquelle sont réglables le diviseur (33) ainsi que les première à quatrième unités (120; 140; 150, 160, 170; 180; 190) via une pluralité de lignes de bus (B1 à B8).

11. Système radiographique avec au moins un dispositif destiné à la production d'images et au traitement d'images,
**caractérisé par** un générateur de rayons X selon l'une quelconque des revendications 3 à 10.
